# EUROPEAN PATENT APPLICATION

(11) **EP 3 973 924 A1**
(43) Date of publication of application: **30.03.2022**
(21) Application number: 21198737.5
(22) Date of filing: 24.09.2021
(51) Int. Cl.: A61F 2/14

(54) **IMPLANT FOR MODIFYING CORNEAL SHAPE AND METHOD OF PREPARING THE SAME**

(30) Priority: 25.09.2020 US 202063083426 P
(71) Applicant: Lions VisionGift, Portland, Oregon 97214 (US)
(72) Inventor: KILIC, Aylin, Portland, 97214 (US)
(74) Representative: Gill Jennings & Every LLP

(57) **Abstract**

Modifying corneal shape involves implantation of a segment of tissue in the corneal stroma, where the properties of the tissue segment and its anatomical placement provide a change in corneal topography. In particular, such methods and materials can be used for correction of defects in corneal shape, such as corneal ectasias.

## Description

### RELATED APPLICATIONS

This application claims priority to United States Provisional Patent Application No. 63/083,426, filed on September 25, 2020, entitled "METHODS AND MATERIALS FOR MODIFYING CORNEAL SHAPE," which is hereby incorporated herein by reference in its entirety.

### TECHNICAL FIELD

The present disclosure relates generally to methods for changing the shape of the cornea and to materials for use in said methods.

### BACKGROUND

The human cornea is an avascular tissue that is generally understood to be responsible for a majority of the eye's refractive power. Therefore, the shape and structure of the cornea, particularly its surface topography, is highly important to the quality of vision. A number of defects in corneal topography have been identified, including corneal ectasias. These non-inflammatory disorders of the eye involve the thinning of the cornea and often result in deformation due to the decrease in structural proteins that normally maintain corneal shape. As the cornea's refractive power is largely a function of its shape, deformation is often accompanied by decreased visual acuity as experienced by persons having these conditions.

Corrective interventions for corneal ectasias have heretofore included corneal collagen cross-linking (CXL), complete or partial corneal transplantation, and device implantation. These approaches, some of which were primarily developed for other indications, have met with varied or questionable success in correcting corneal topography. A number of post-operative complications are known to arise from intrastromal corneal ring segment (ICRS) implantation in particular, including thinning of the corneal epithelium, stromal dissolution, and eventual corneal perforation.

### BRIEF DESCRIPTION OF THE DRAWINGS

The embodiments disclosed herein will become more fully apparent from the following description and appended claims, taken in conjunction with the accompanying drawings. The patent or application file contains at least one drawing executed in color. Copies of this patent or patent application publication with color drawings will be provided by the Office upon request and payment of the necessary fee.
FIG. 1 is a photograph of an implanted prior art corneal implant device, illustrating an adverse effect known to be associated with such devices.
FIG. 2 shows two diagrams illustrating the structure and conventional use of prior art corneal implant devices.
FIG. 3A is a cross-sectional image of a cornea after conventional implantation of a prior art corneal implant device, illustrating the epithelial thinning that can arise from typical placement of such devices.
FIG. 3B is a cross-sectional image of another cornea after conventional implantation of a prior art corneal implant device, illustrating the epithelial thinning that can arise from typical placement of such devices.
FIG. 3C shows frontal and cross-sectional images (top section) of a cornea implanted with a prior art device.
FIG. 3D shows a whole corneal thickness (pachymetry) map (center) and an epithelial thickness map (right) and associated data (left) for the post-operative cornea shown in FIG. 3C.
FIG. 4 illustrates commencement of insertion of a tissue segment through an incision on the corneal surface and into an intrastromal channel created in accordance with the present disclosure.
FIG. 5A is an image illustrating the effects of implantation of a prior art device on corneal shape.
FIG. 5B is an image illustrating the shape of a cornea implanted with a tissue segment in accordance with an embodiment.
FIG. 6A shows topographic maps of a cornea before and after implantation of a tissue segment in accordance with the present disclosure, and a map showing the resulting topographical change.
FIG. 6B shows topographic maps of the cornea shown in FIG. 6A, before removal (top row) and after reimplantation and relocation (bottom row) of the tissue segment.
FIG. 7 shows topographic maps of a cornea before and after implantation of a tissue segment in accordance with the present disclosure, and the resulting topographical change.
FIG. 8 shows topographic maps of a cornea with associated data before and after implantation of a tissue segment in accordance with the present disclosure, and the resulting topographical change.
FIG. 9 is a plot of post-operative uncorrected distance visual acuity (UCDVA) vs pre-operative UCDVA for 14 eyes in which tissue segment implantations were performed according to the present disclosure.
FIG. 10 is a plot of post-operative vs pre-operative mean keratotomy values (KMEAN) for nine corneas in which tissue segment implantations were performed according to the present disclosure.
FIG. 11 shows a photograph of a tissue segment shaped in accordance with an embodiment of the present disclosure.
FIG. 12 provides a side-by-side comparison of an eye implanted with a prior art device (left photo) and an eye implanted with a tissue segment in accordance with the present disclosure (right photo).
FIG. 13 is a post-recovery photograph of an eye in which a tissue segment was implanted according to the present disclosure.

### DETAILED DESCRIPTION

The present disclosure is directed to methods and therapeutic substances for modifying the shape of the cornea of the eye, particularly for correcting defects in corneal surface topography.

A substance for use in treating a defect in or otherwise correcting corneal topography in accordance with the present disclosure can comprise a segment of tissue configured for placement in the stroma of a cornea so as to produce a topographical change in the cornea. The tissue can be optically transparent so as to transmit light having wavelengths in the visible spectrum. In various embodiments, the tissue is corneal tissue.

More particularly, the tissue can be an allograft, i.e., sourced from an individual other than the subject. In some embodiments, the tissue segment comprises fresh corneal tissue, such as may have been recently removed from the cornea of a human donor or compatible animal. Accordingly, preparing an implant for such a use can comprise obtaining a sample of a tissue sourced from a donor and processing the sample to produce a segment of tissue configured for placement in the stroma of a cornea. In some embodiments, the tissue segment comprises corneal tissue that has been processed and preserved to remain stable until implantation. Such processing can include sterilizing the tissue. Sterilization methods including, but not limited to, irradiation sterilization using e.g., gamma rays or electron beam can be employed. Corneal tissue can be preserved and stored for use in accordance with the present disclosure by various techniques, including organ culture and hypothermic storage. In some embodiments the tissue segment can be provided in a preservative agent. In certain embodiments, the preservative agent comprises glycerin. In certain embodiments the tissue is stable at ambient temperatures from about 2 °C to about 40 °C.

Providing a segment of tissue for use as described herein can further comprise sectioning a tissue sample to produce a selected shape with selected dimensions. The shape and dimensions of the tissue segment can be selected in consideration of a number of factors, including the size of the cornea as a whole, the nature of topographical change to be achieved, and the dimensions of the surgical space into which the tissue segment is to be implanted. In an aspect, the dimensions of the segment can affect the topographical change resulting from implantation. For example, the degree of change may increase with increasing width and/or thickness of the segment.

In various embodiments, the tissue segment has a generally oblong shape, which can further be substantially linear or substantially arcuate. In some embodiments, the segment has a width of from about 1 mm to about 4 mm, or more particularly from about 2 mm to about 3 mm. In some embodiments, the segment has a thickness of about 50 microns to about 550 microns, or more particularly from about 70 microns to about 400 microns, or from about 100 microns to about 250 microns, or from about 70 microns to about 170 microns, or from about 150 microns to about 250 microns, or from about 220 microns to about 320 microns, or from about 300 microns to about 400 microns, or from about 380 microns to about 480 microns, or from about 450 microns to about 550 microns. In some embodiments, the shape of the tissue segment can vary in width and/or thickness along its length. In some embodiments, this variation can be irregular or random. In other embodiments, the width and/or thickness can vary from one end of the segment to the other according to a selected pattern. In certain embodiments, the segment has a first end and a second end that differ from each other in one or more dimensions. In more particular embodiments, the second end has a greater width, a greater thickness, or both as compared to the first end. For example, the first end can have a width of from about 1 mm to about 3 mm and the second end can have a width of from about 2 mm to about 4 mm and that is greater than the width of the first end.

In some embodiments the tissue segment has a generally arcuate shape. In an aspect, the radius of curvature and the arc length of the segment can be selected in consideration of the space in which the segment will reside once implanted. In some embodiments, the tissue segment can have an arc length such that the segment subtends an angle of from about 90 degrees to about 270 degrees. In particular embodiments, the angle is from about 100 degrees to about 200 degrees, or from about 120 degrees to about 190 degrees. In an aspect, the arc length of the segment can affect the topographical change resulting from implantation. For example, the degree of topographical change may increase with increasing arc length of the segment.

A method for correcting corneal topography in a subject can comprise the steps of identifying a surgery site on a surface of a subject's cornea, or more or particularly for making an initial incision; making the incision through the surface at the selected incision site and into a stroma of the cornea, and then creating a channel within the stroma extending from the incision at a depth beneath the surface; and then inserting a segment of a tissue into the channel to achieve a topographical change in the cornea.

In various embodiments, the corrective procedure described herein involves creating a channel in the corneal stroma to accommodate a tissue segment implant. This is initiated by making an incision at a selected site on the corneal surface, where said incision extends through the corneal epithelium and into the corneal stroma. In an aspect, the incision can extend into the stroma to a selected depth below the surface, for example the depth at which the implant is to reside.

The procedure continues by creating a channel that extends through the stroma from the incision site. In some embodiments, the implanted tissue will reside between two lamellae of the stroma. Accordingly in some embodiments, the channel can be introduced between lamellae. In particular embodiments, substantially the entire channel is situated between two lamellae. In other embodiments, the channel may pass through one or more lamellae. In some embodiments, this step involves creating most or all of the channel before inserting the tissue segment therein. In some embodiments, at least a portion of the channel is created by insertion of the tissue. The channel can be created using surgical tools suitable for such a task, for example tools for delaminating lamellae such as surgical hooks (e.g. pocketing hooks and Sinskey hooks), surgical spatulas such as Suarez spreaders, and glides. In some embodiments, laser surgical techniques can be used in creating the channel.

Conventional methods using intrastromal implant devices can involve implanting two arcuate pieces of synthetic material (e.g., paired ICRS) among the deeper layers of the stroma, where the two devices are situated on opposite sides of the central visual zone. In accordance with various embodiments discussed herein, a method of correcting corneal topography involves the creation of a single channel, most of which involves one semicircle of the cornea.

In some embodiments, the channel can be formed so as to be situated mostly or completely outside the central optical zone. More particularly, at least a portion of the channel can involve one or more of the paracentral zone, peripheral zone, or limbus. In some embodiments, the channel is tangential to the central optical zone and is substantially linear or substantially arcuate in shape. In various embodiments, the channel is formed to have a substantially arcuate shape so as to curve around the central optical zone. In some embodiments, an arcuate channel can have an arc length such that the channel subtends an angle of from about 90 degrees to about 270 degrees. In particular embodiments, the angle is from about 100 degrees to about 200 degrees, or from about 120 degrees to about 190 degrees.

Conventional approaches to corneal correction with intrastromal device implantation involve implanting such devices in the deeper layers of the stroma. In fact, prevailing practice suggests that achieving maximal depth is important so as to avoid inducing astigmatism and to lessen disruption of the corneal epithelium. In contrast, the inventors have unexpectedly found that the use of superficially situated tunnels in the methods disclosed herein are more effective in creating beneficial changes in corneal topography. The inventors have further found that the methods herein result in markedly less post-operative thinning of the epithelium. Accordingly, in various embodiments, the channel is created at a selected depth of less than 200 microns below the corneal surface. In particular embodiments, the depth is from about 60 microns to about 150 microns, or from about 80 microns to about 125 microns, or from about 90 microns to about 110 microns.

The dimensions of the channel can be selected in consideration of a number of factors, including the size of the cornea as a whole, the nature of topographical change to be achieved, and the dimensions of the tissue implant. In some embodiments, the channel is formed to comprise an arc having a selected inner diameter, where the inner diameter can be from about 3 mm to about 5 mm. In some embodiments, the channel is formed to comprise an arc having a selected outer diameter, where the outer diameter can be from about 7 mm to about 9 mm.

In accordance with the above embodiments, a further step in the procedure involves inserting a segment of tissue into the channel via the incision. This can involve grasping one end of the segment, inserting it into the channel via the incision, and then moving the segment along the length of the channel until the desired placement is achieved. In some embodiments, where one end of the segment is smaller in width and/or thickness than the other, the smaller end can be introduced into the channel first. Once the segment is in place, the incision may be closed by techniques known to be suited for effective post-operative healing of the cornea.

In some embodiments, the dimensions of the tissue segment can be selected to provide a particular relationship to the dimensions of the channel. For example, the tissue segment can be shaped to be slightly smaller than the channel to provide ease of insertion, while a shape that is slightly larger than the channel may be selected to better filling of the channel space. In another aspect a tissue segment may be selected that is either slightly larger or slightly smaller than the channel to provide particular post-operative results with regard to topographical change, visual acuity, or healing. In some embodiments, the tissue segment has a width that is from about 75% to about 110% the width of the channel.

Implantation of a tissue segment in accordance with the various embodiments discussed herein can produce a change in the topography of the cornea. More particularly, an aspect of the embodiments is to provide for treatment of a defect in corneal topography. Such defects can include corneal ectasias in which thinning of the cornea results in abnormal corneal shapes. Such ectasias include, but are not limited to, keratoconus, keratoglobus, pellucid marginal degeneration, as well as post-surgical ectasias. In certain embodiments, the defect treated is keratoconus, in which the cornea develops a cone shape. In some embodiments, the topographical change produced by implantation is an increase in the thickness of the cornea, particularly in the location of the implant. In some embodiments, the topographical change comprises a decrease in curvature of the cornea.

The shape of the cornea directly affects its refractive properties. For example, an increase in curvature in an area of the cornea causes the focal point of the cornea to shift, producing refractive error and decreasing visual acuity. A topographical change that includes flattening of the affected area can thereby also produce a change in the local or overall refractive properties of the cornea. An aspect of the embodiments described herein is that implantation of the tissue segment does not have a negative effect on visual acuity. In some embodiments, the topographical change results in no loss in visual acuity. In particular embodiments, the topographical change produces an increase in a particular measure of visual acuity, such as uncorrected visual acuity (UCVA) or uncorrected distance visual acuity (UCDVA).

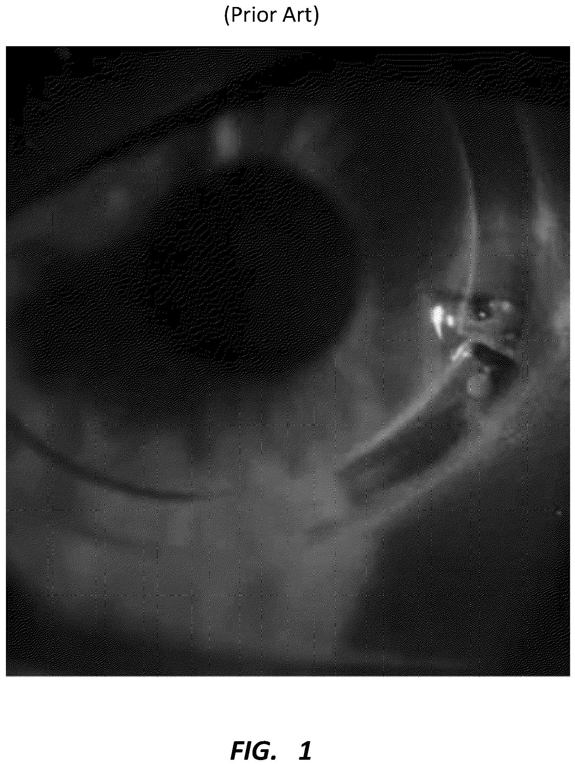

A number of complications and other negative outcomes have been observed with conventional approaches to corneal reshaping, particularly the use of paired intrastromal corneal ring segments (ICRS). For example, implantation of typical ICRS often results in thinning of the corneal epithelium over the implantation site. It should be noted that minimizing disruption to the epithelium is one rationale for the conventional approach of implanting ICRS in the posterior stroma. In contrast, an unexpected aspect of the present disclosure is that superficial implantation of a tissue segment according to the embodiments herein produces little to no thinning of the corneal epithelium. ICRS implantation also can result in dissolution of the stroma and the epithelium, often to the degree that ulceration occurs, as shown in FIG. 1. In contrast, such outcomes can be avoided with implantation of a tissue segment according to the embodiments herein. Furthermore, unlike ICRS, which are often visible to an observer looking at the treated eye, the tissue segments described herein are much less visible and thereby can be more desirable from a cosmetic standpoint.

In accordance with the present disclosure, methods of correcting corneal topography can further comprise a preliminary step of selecting parameters of the implantation procedure so as to produce a topographical change. Such parameters can include the incision site, channel location, channel dimensions, and tissue segment dimensions. In some embodiments, the selection can be made based on measurements of the cornea taken pre-operatively. These measurements include measurements of corneal topography such as curvature, thickness, and elevation.

A number of available approaches and techniques for assessing corneal topography can be employed. In particular, assessing corneal topography can involve imaging the cornea and creating a curvature map of the anterior surface of the cornea (and in some cases, the posterior surface as well). Various methods can be employed to convert curvature in such maps into refractive power values, and color coding can be used to provide a quantitative visual representation of relief. Such maps allow one to visualize the contours, elevation, and steepness of corneal deformations. In accordance with the embodiments herein, such maps can be used to identify one or more areas of the cornea that exhibit an abnormal degree of curvature. Similar imaging techniques can be used to provide a map of corneal thickness, and such maps can be used to identify areas of abnormal corneal thinness. The information provided by such maps can be used to identify a topographical change that will improve corneal shape, such as flattening of a steep area of the cornea and/or increasing symmetry in the cornea's shape. Based on this assessment, various surgical parameters can be selected for implanting a tissue segment as described above so as to produce the topographical change. In a particular example, a steep axis of the cornea can be selected for the incision site to facilitate effective channel placement and insertion of a tissue segment. In another example, the incision site can be selected based upon the location of a particular corneal deformation, such as a cone-shaped protrusion of the cornea.

In addition to corneal mapping, other measurements and assessments including UCVA, UCDVA, best spectacle-corrected visual acuity (BSCVA), manifest refraction, and endothelial cell density measurement may be employed in selecting surgical parameters. In certain embodiments, pre-operative assessments can be incorporated into a nomogram which can be used to match surgical parameters with topographical changes that provide particular post-operative outcomes. In various such embodiments, a nomogram can correlate channel depth, tissue segment width, tissue segment thickness, or a combination thereof with the effectiveness of implantation. For example, increased effectiveness can be correlated with decreased channel depth, increased tissue segment width, increased tissue segment thickness, or combinations thereof.

### EXAMPLES

### Comparative Example - Prior Art Implant Devices

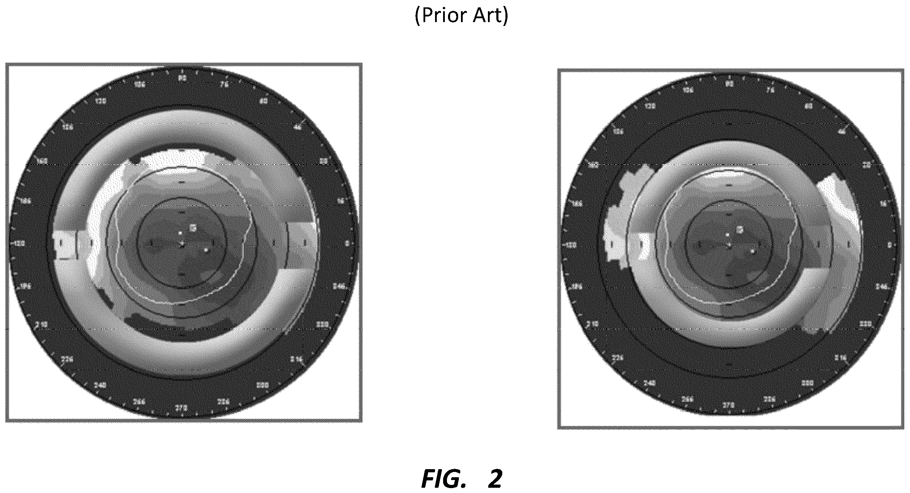

Conventional intrastromal corneal ring segment involves implantation of paired ring segments in the deep layers of the stroma on opposite sides of the cornea. FIG. 2 shows two corneal topographic maps illustrating a typical surgical arrangement of paired ICRSs (indicated by arrows).

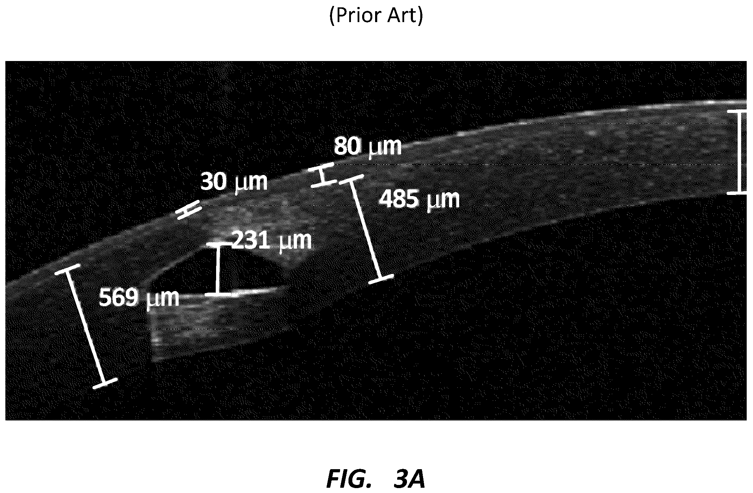

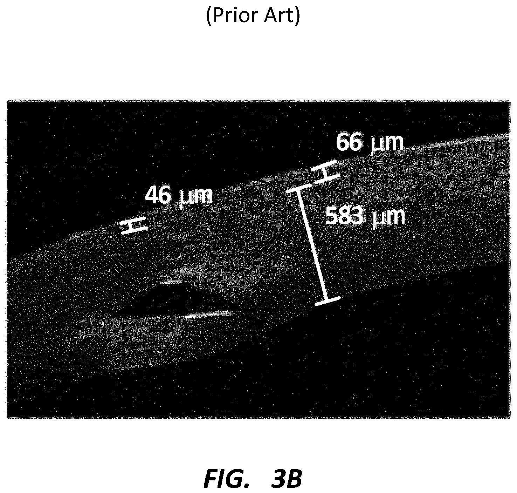

FIGs. 3A through 3C present cross-sectional images of corneas after conventional implantation of existing ICRSs, showing the epithelial thinning resulting from typical placement of these devices. For example, in FIG. 3A the corneal epithelium directly over the plastic implant (appears in cross-section as a triangular dark region) is only 30 microns thick, as compared to the 80-micron-thick epithelium in other regions. In another example as shown in FIG. 3B, the corneal epithelium directly over the implant is 46 microns thick, as compared to the 66-micron-thick epithelium in other regions.

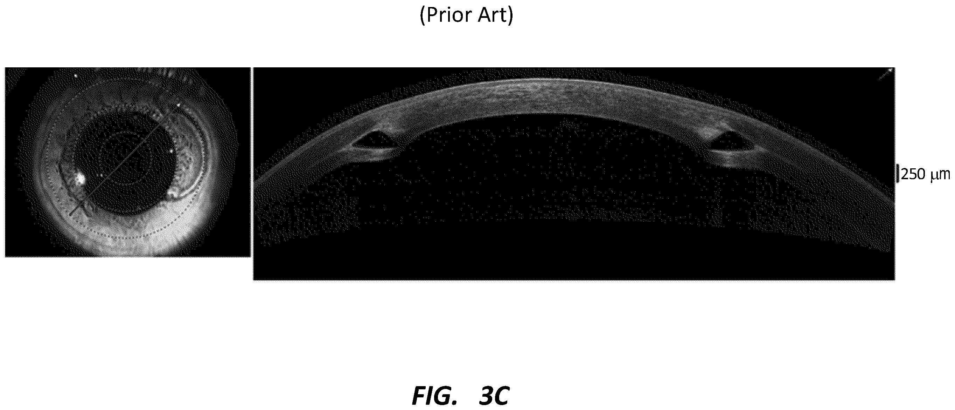

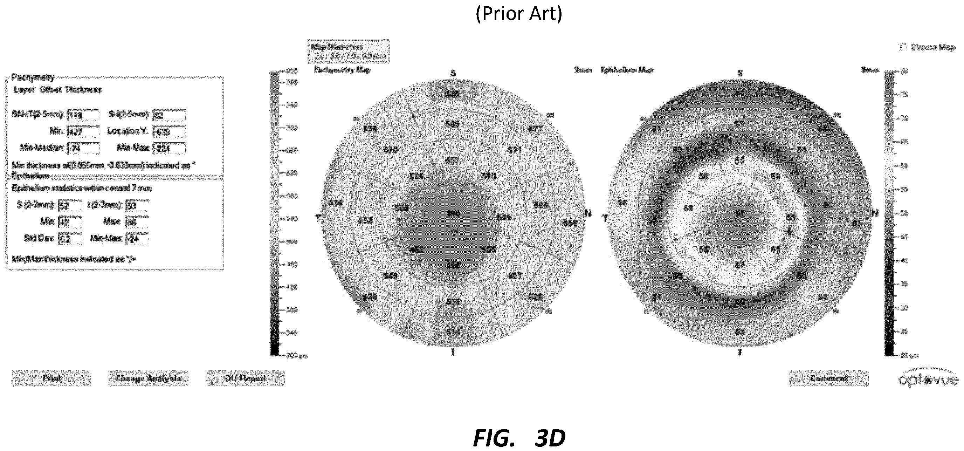

FIG. 3D shows a whole corneal thickness (pachymetry) map (center) and an epithelial thickness map (right) for the cornea implanted with an ICRS shown in FIG. 3C. Color coding of thickness is indicated by the adjacent scale bars. The epithelial thickness map shows a roughly circular area of epithelial thinning (blue and dark green) corresponding to the location of the implanted ICRSs.

### Examples of Benefits of the Present Disclosure

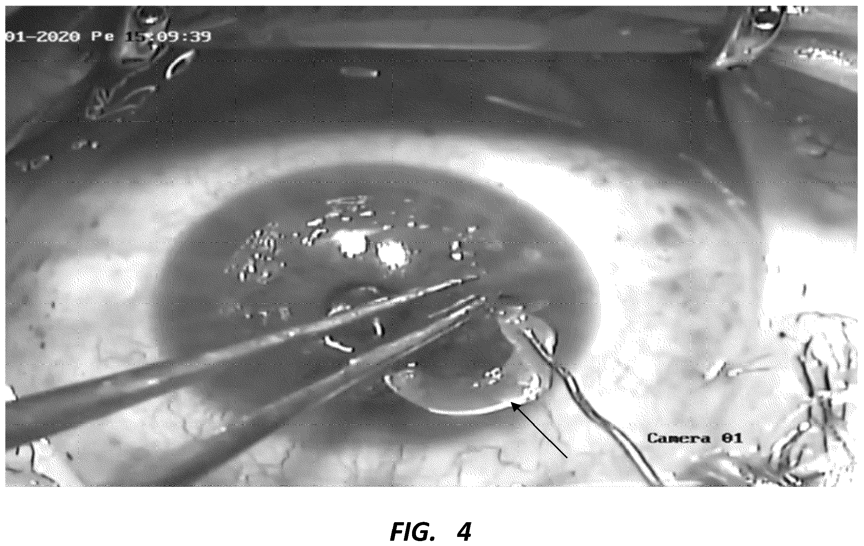

FIG. 4 shows the commencement of insertion of a tissue segment (indicated by the arrow) into an intrastromal channel created in accordance with the present disclosure.

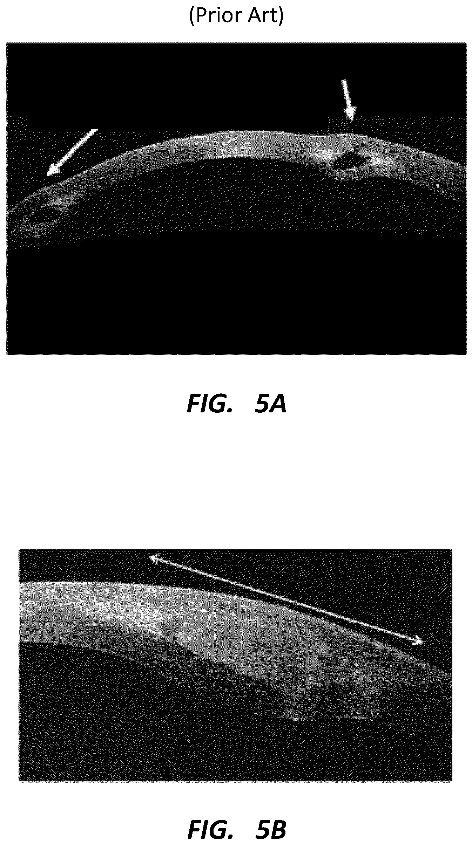

FIG. 5A and FIG. 5B respectively show the comparative effects on corneal shape of implantation of an ICRS and a tissue segment of the present disclosure. As indicated by arrows in FIG. 5A, significant deformations ("bumps") of the corneal surface are evident after implantation of an ICRS. In contrast, implantation of a tissue segment of the present disclosure (FIG. 5B) produces little to no deformation of the corneal surface, despite its comparatively superficial placement within the stroma.

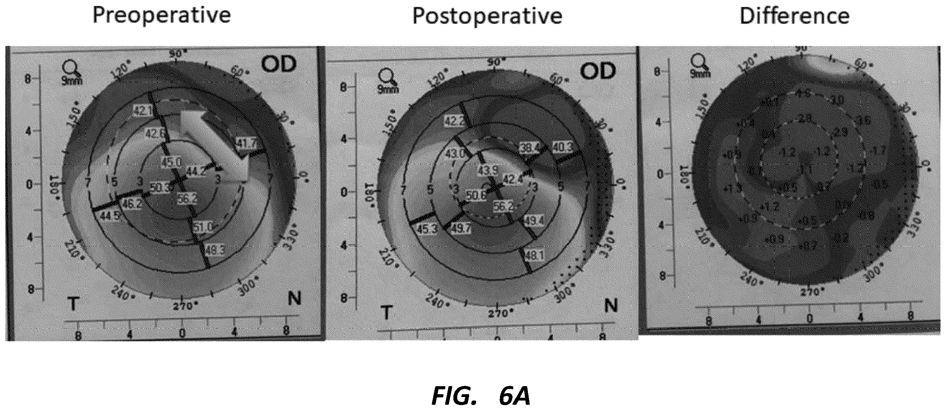

FIG. 6A through FIG. 8 each show topographic maps of a cornea before ("Preoperative") and after ("Postoperative") implantation of a tissue segment of the present disclosure, and the resulting topographical change ("Difference"). In each map, curvature values are expressed in diopters (D) and represented by a color code in which blue = flatter and red = steeper. The arrow represents the axis on which the incision site and channel is located.

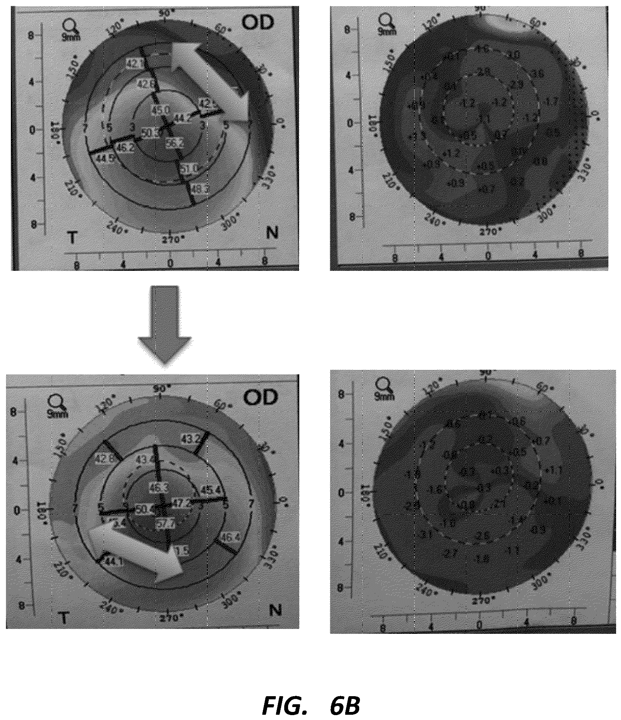

FIG. 6A and FIG. 6B show the results of two successive tissue segment implantation procedures on the same cornea and illustrate the role of implant site selection in achieving beneficial topographical change. In FIG. 6A a steeply curved bulge can be seen between approximately 165 degrees and 345 degrees of the "Preoperative" map. Tissue segment implantation along the indicated axis resulted in flattening in other areas of the cornea while the localized bulge remained, as shown in the "Postoperative" and "Difference" maps. However, removal and reimplantation of the tissue segment produced an improved corneal shape as shown in the postoperative (lower left, with reimplantation axis indicated therein) and topographical change (lower right) maps in FIG. 6B. The preoperative map and topographical change maps from FIG. 6A are also reproduced in the upper row of FIG. 6B for comparison. In addition to improved shape, the eye exhibited improved UCVA (0.6) after reimplantation as compared to the post-operative UCVA for the prior procedure (0.16).

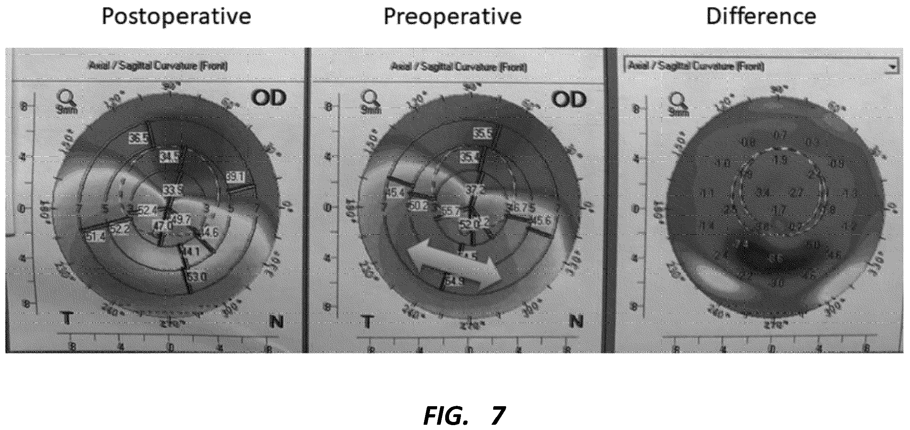

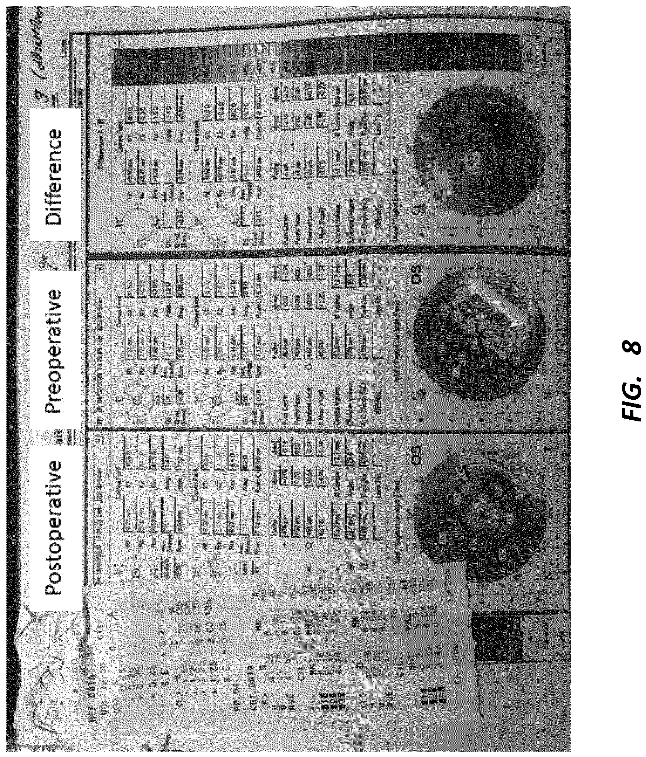

FIG. 7 shows topographical maps for another cornea implanted with a tissue segment in accordance with the present disclosure, in which the pre-operative UCVA (0.1) was improved to a post-operative value of 0.7. FIG. 8 shows both topographical data and maps for another corneal implantation procedure in accordance with the present disclosure.

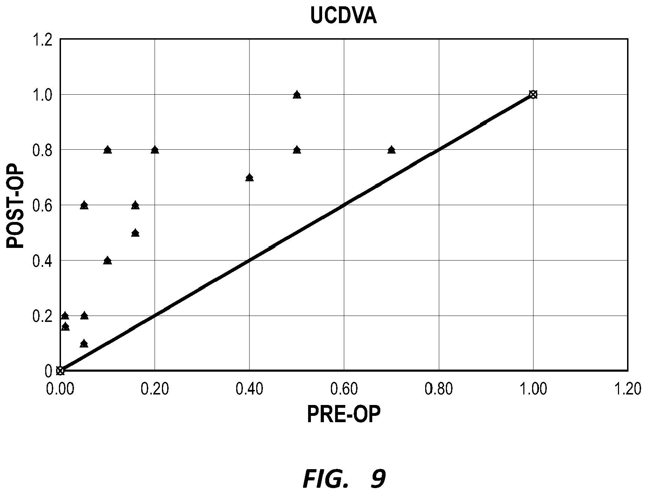

A plot of post-operative uncorrected distance visual acuity (UCDVA) vs pre-operative UCDVA for 14 eyes in which tissue segment implantations were performed according to the present disclosure is shown in FIG. 9, along with the identity line for comparison. As shown, each eye showed varying degrees of post-operative improvement in acuity, with none exhibiting a loss in UCDVA.

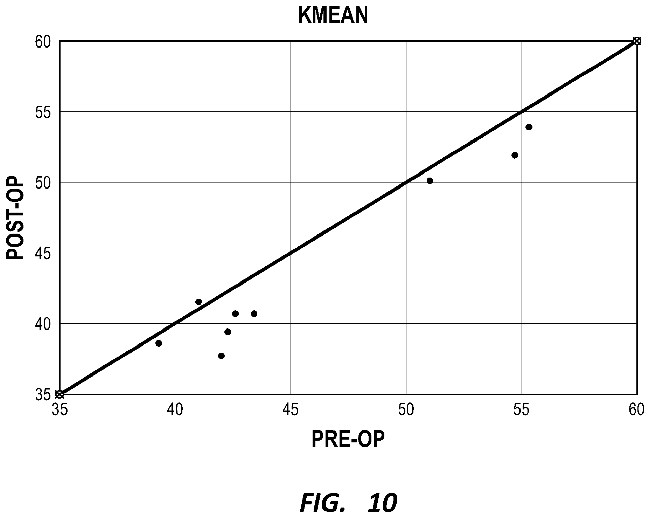

A plot of post-operative vs pre-operative mean keratotomy values (KMEAN) for nine corneas in which tissue segment implantations were performed according to the present disclosure is shown in FIG. 10, along with the identity line for comparison. As shown, eight of the nine corneas exhibited a post-operative decrease in mean curvature, with the remaining cornea exhibiting a slight increase.

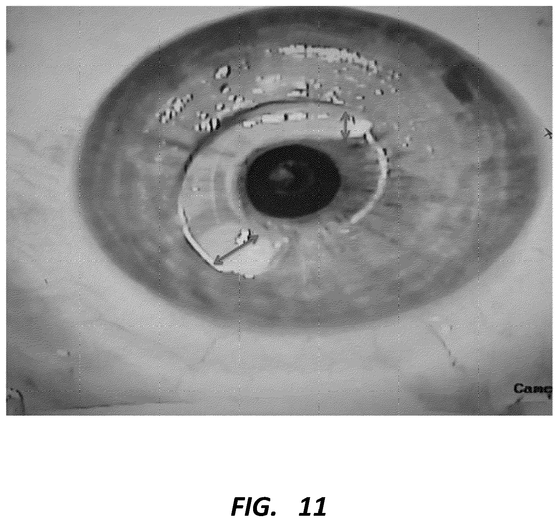

In some cases, a particular segment shape may facilitate implantation and/or topographical change. FIG. 11 shows a photograph of a tissue segment in accordance with an embodiment of the present disclosure, in which the two ends of the segment have different widths, as indicated by the arrows.

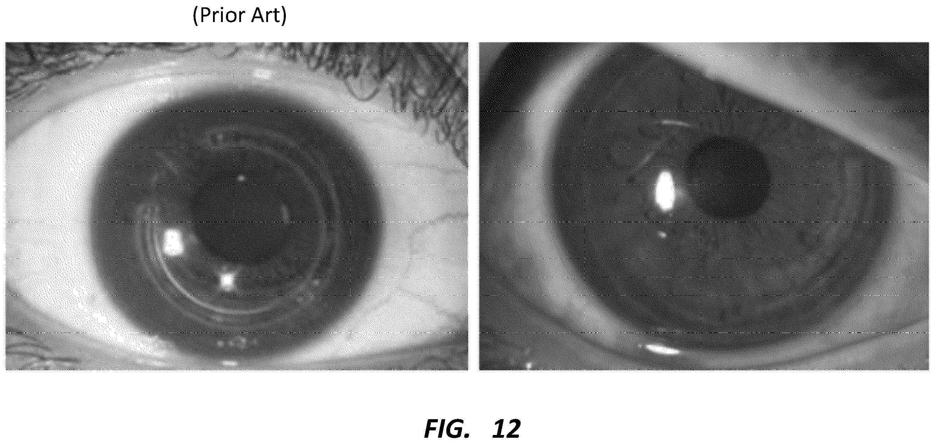

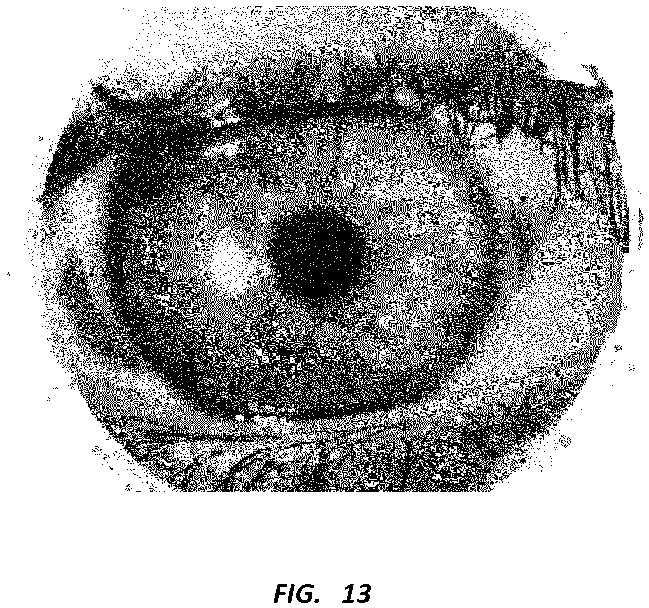

Corneal correction using materials and procedures described herein can also provide a more pleasing post-operative appearance than prior art approaches. FIG. 12 provides a side-by-side comparison of an eye implanted with a conventional ICRS (left photo) and an eye implanted with a tissue segment in accordance with the present disclosure (right photo). A post-operative photograph of another eye in which a tissue segment was implanted according to the present disclosure is shown in FIG. 13.

Any methods disclosed herein comprise one or more steps or actions for performing the described method. The method steps and/or actions may be interchanged with one another. In other words, unless a specific order of steps or actions is required for proper operation of the embodiment, the order and/or use of specific steps and/or actions may be modified.

References to approximations are made throughout this specification, such as by use of the terms "substantially" and "about." For each such reference, it is to be understood that, in some embodiments, the value, feature, or characteristic may be specified without approximation. For example, where qualifiers such as "about" and "substantially" are used, these terms include within their scope the qualified words in the absence of their qualifiers. All ranges also include both endpoints.

It will be apparent to those having skill in the art that many changes may be made to the details of the above-described embodiments without departing from the underlying principles of the invention. The scope of the present invention should, therefore, be determined only by the following claims.

### Numbered Embodiments:

Embodiment 1. A method of correcting corneal topography in a subject, comprising:
   identifying an incision site on a surface of a cornea of the subject;
   making an incision through the surface at the incision site and into a stroma of the cornea;
   creating a channel within the stroma extending from the incision at a depth beneath the surface; and
   inserting a segment of tissue into the channel to achieve a topographical change in the cornea,
   wherein the segment of tissue is sterile.
Embodiment 2. The method of embodiment 1, wherein the segment of tissue is optically transparent.
Embodiment 3. The method of embodiment 1 or embodiment 2, wherein the segment of tissue is corneal tissue.
Embodiment 4. The method of embodiment 3, wherein the corneal tissue is an allograft.
Embodiment 5. The method of any of embodiments 1-4, wherein the topographical change is an increase in a thickness of the cornea, a decrease in a curvature of the cornea, a change in a refractive property of the cornea, an increase in symmetry of the cornea, or combinations thereof.
Embodiment 6. The method of any of embodiments 1-5, wherein the topographical change results in no loss in visual acuity.
Embodiment 7. The method of any of embodiments 1-6, wherein creating the channel comprises creating a single channel in the cornea of the subject.
Embodiment 8. The method of embodiment 7, wherein inserting the segment of tissue into the channel comprises inserting a single segment of the tissue into the single channel.
Embodiment 9. The method of any of embodiments 1-8, wherein the depth is less than about 200 microns.
Embodiment 10. The method of embodiment 9, wherein the depth is from about 60 microns to about 150 microns.
Embodiment 11. The method of any of embodiments 1-10, wherein the channel has an arcuate shape subtending an angle of from about 90 degrees to about 270 degrees.
Embodiment 12. The method of embodiment 11, wherein the angle is from about 100 degrees to about 200 degrees.
Embodiment 13. The method of embodiment 11 or embodiment 12, wherein the arcuate shape has an inner diameter from about 3 mm to about 5 mm.
Embodiment 14. The method of embodiment 11 or embodiment 12, wherein the arcuate shape has an outer diameter from about 7 mm to about 9 mm.
Embodiment 15. The method of any of embodiments 1-14, wherein the segment of tissue has an arcuate shape subtending an angle of from about 90 degrees to about 270 degrees.
Embodiment 16. The method of embodiment 15, wherein the angle is from about 100 degrees to about 200 degrees.
Embodiment 17. The method of any of embodiments 1-16, wherein the segment of tissue has a width from about 1 mm to about 4 mm.
Embodiment 18. The method of embodiment 17, wherein the segment of tissue has a width from about 2 mm to about 3 mm.
Embodiment 19. The method of any of embodiments 1-16, wherein the segment of tissue has a first end and a second end, and wherein a width of the second end is greater than a width of the first end.
Embodiment 20. The method of embodiment 19, wherein the width of the first end is from about 1 mm to about 2 mm.
Embodiment 21. The method of embodiment 19, wherein the width of the second end is from about 2 mm to about 4 mm.
Embodiment 22. The method of any of embodiments 19-21, wherein the inserting step is commenced by inserting the first end into the channel.
Embodiment 23. The method of any of embodiments 1-22, wherein the segment of tissue has a width that is from about 75% to about 110% of a width of the channel.
Embodiment 24. The method of any of embodiments 1-23, wherein the segment of tissue has a thickness of about 50 microns to about 550 microns.
Embodiment 25. The method of any of embodiments 1-24, wherein the incision site is selected based on one or more preoperative measurements of the cornea.
Embodiment 26. The method of embodiment 25, wherein the one or more preoperative measurements are selected from curvature, thickness, elevation, and combinations thereof.
Embodiment 27. The method of any of embodiments 1-24, wherein the incision site is on a steep axis of the cornea.
Embodiment 28. The method of any of embodiments 1-24, wherein the incision site is selected based on a location of a deformity of the cornea.
Embodiment 29. A substance for use in treating a defect in corneal topography, comprising a segment of tissue configured for placement within a stroma of a cornea having the defect so as to produce a topographical change in said cornea, wherein the tissue is sterile.
Embodiment 30. The substance of embodiment 29, wherein the tissue is optically transparent.
Embodiment 31. The substance of embodiment 29 or embodiment 30, wherein the tissue is corneal tissue.
Embodiment 32. The substance of embodiment 31, wherein the corneal tissue is preserved in a solution comprising glycerin.
Embodiment 33. The substance of embodiment 29, wherein the tissue is stable at ambient temperatures from about 20 °C to about 30 °C.
Embodiment 34. The substance of any of embodiments 29-33, wherein the segment of tissue has a thickness of from about 50 microns to about 550 microns.
Embodiment 35. The substance of any of embodiments 29-34, wherein the segment of tissue has an arcuate shape subtending an angle of from about 90 degrees to about 270 degrees.
Embodiment 36. The substance of embodiment 35, wherein the angle is from about 100 degrees to about 200 degrees.
Embodiment 37. The substance of any of embodiments 29-36, wherein the segment of tissue has a width from about 1 mm to about 4 mm.
Embodiment 38. The substance of embodiment 37, wherein the segment of tissue has a width from about 2 mm to about 3 mm.
Embodiment 39. The substance of any of embodiments 29-36, wherein the segment of tissue has a first end and a second end, and wherein a width of the second end is greater than a width of the first end.
Embodiment 40. The substance of embodiment 39, wherein the width of the first end is from about 1 mm to about 2 mm.
Embodiment 41. The substance of embodiment 39, wherein the width of the second end is from about 2 mm to about 4 mm.
Embodiment 42. The substance of any of embodiments 29-41, wherein the defect is a corneal ectasia selected from keratoconus, keratoglobus, pellucid marginal degeneration, or post-surgical ectasia.
Embodiment 43. The substance of any of embodiments 29-42, wherein the topographical change is an increase in a thickness of the cornea, a decrease in a curvature of the cornea, a change in a refractive property of the cornea, an increase in symmetry of the cornea, or combinations thereof.
Embodiment 44. The substance of any of embodiments 29-43, wherein the segment of tissue is configured for placement within a channel created in the stroma of the cornea.
Embodiment 45. A method of preparing an implant for correcting corneal topography, comprising:
   obtaining a sample of a tissue;
   sectioning the sample to produce a segment of tissue having a shape selected for placement within a stroma of a cornea, wherein the shape is oblong and includes a first end and a second end.
Embodiment 46. The method of embodiment 45, wherein the tissue is optically transparent.
Embodiment 47. The method of embodiment 45 or embodiment 46, wherein the tissue is corneal tissue.
Embodiment 48. The method of any of embodiments 45-47, further comprising sterilizing the tissue.
Embodiment 49. The method of embodiment 48, wherein the sterilizing is performed by irradiation.
Embodiment 50. The method of embodiment 49, wherein the irradiation is selected from gamma irradiation or electron beam irradiation.
Embodiment 51. The method of any of embodiments 45-50, wherein the shape is arcuate and subtends an angle of from about 90 degrees to about 270 degrees.
Embodiment 52. The method of any of embodiments 45-51, wherein the segment of tissue has a width from about 1 mm to about 4 mm.
Embodiment 53. The method of any of embodiments 45-51, wherein a width of the second end is greater than a width of the first end.
Embodiment 54. The method of embodiment 53, wherein the width of the first end is from about 1 mm to about 2 mm.
Embodiment 55. The method of embodiment 53, wherein the width of the second end is from about 2 mm to about 4 mm.
Embodiment 56. The method of any of embodiments 45-55, further comprising placing the segment into a preservative composition.
Embodiment 57. The method of embodiment 56, wherein the preservative composition comprises glycerin.

## Claims

1. A substance for use in the treatment of a defect in corneal topography, wherein the substance comprises a segment of tissue configured for placement within a stroma of a cornea having the defect so as to produce a topographical change in said cornea, wherein the tissue is sterile.

2. The substance for use of claim 1, wherein the tissue is optically transparent; and/or
wherein the tissue is corneal tissue, preferably wherein the corneal tissue is preserved in a solution comprising glycerin.

3. The substance for use of claim 1, wherein the tissue is stable at ambient temperatures from about 20 °C to about 30 °C.

4. The substance for use of any of claims 1-3, wherein the segment of tissue has a thickness of from about 50 microns to about 550 microns; and/or
wherein the segment of tissue has an arcuate shape subtending an angle of from about 90 degrees to about 270 degrees, preferably wherein the angle is from about 100 degrees to about 200 degrees.

5. The substance for use of any of claims 1-4, wherein the segment of tissue has a width from about 1 mm to about 4 mm, preferably wherein the segment of tissue has a width from about 2 mm to about 3 mm.

6. The substance for use of any of claims 1-5, wherein the segment of tissue has a first end and a second end, and wherein a width of the second end is greater than a width of the first end, preferably
wherein the width of the first end is from about 1 mm to about 2 mm or the width of the second end is from about 2 mm to about 4 mm.

7. The substance for use of any of claims 1-6, wherein the defect is a corneal ectasia selected from keratoconus, keratoglobus, pellucid marginal degeneration, or post-surgical ectasia; and/or
wherein the topographical change is an increase in a thickness of the cornea, a decrease in a curvature of the cornea, a change in a refractive property of the cornea, an increase in symmetry of the cornea, or combinations thereof; and/or
wherein the segment of tissue is configured for placement within a channel created in the stroma of the cornea.

8. The substance for use of any of claims 1-7, wherein the use comprises:
identifying an incision site on a surface of a cornea of a subject;
making an incision through the surface at the incision site and into a stroma of the cornea;
creating a channel within the stroma extending from the incision at a depth beneath the surface; and
inserting the segment of tissue into the channel to achieve a topographical change in the cornea.

9. A method of preparing an implant for correcting corneal topography, comprising:
obtaining a sample of a tissue;
sectioning the sample to produce a segment of tissue having a shape selected for placement within a stroma of a cornea, wherein the shape is oblong and includes a first end and a second end.

10. The method of claim 9, wherein the tissue is optically transparent; and/or wherein the tissue is corneal tissue.

11. The method of claim 9 or 10, further comprising sterilizing the tissue, preferably wherein the sterilizing is performed by irradiation, preferably wherein the irradiation is selected from gamma irradiation or electron beam irradiation.

12. The method of any of claims 9-11, wherein the shape is arcuate and subtends an angle of from about 90 degrees to about 270 degrees.

13. The method of any of claims 9-12, wherein the segment of tissue has a width from about 1 mm to about 4 mm.

14. The method of any of claims 9-12, wherein a width of the second end is greater than a width of the first end, preferably
wherein the width of the first end is from about 1 mm to about 2 mm or the width of the second end is from about 2 mm to about 4 mm.

15. The method of any of claims 9-14, further comprising placing the segment into a preservative composition, preferably wherein the preservative composition comprises glycerin.
